(19) Europäisches Patentamt
European Patent Office
Office européen des brevets

(11) EP 0 926 130 B1

(12) EUROPÄISCHE PATENTSCHRIFT

(45) Veröffentlichungstag und Bekanntmachung des Hinweises auf die Patenterteilung:
**12.06.2002 Patentblatt 2002/24**

(51) Int Cl.[7]: **C07C 209/48**, C07C 209/26, C07C 209/52

(21) Anmeldenummer: **98120710.3**

(22) Anmeldetag: **31.10.1998**

# (54) Verfahren zur Herstellung von 3-Aminomethyl-3,5,5-trimethyl-cyclohexylamin mit einem cic/trans-Isomerenverhältnis von mindestens 70/30

Process for the preparation of 3-aminomethyl-3,5,5-trimethyl-cyclohexylamine with cis/trans-Isomer ratio of at least 70/30

Procédé pour la préparation de 3-aminomethyl-3,5,5-trimethyl-cyclohexylamine avec un rapport d'isomères cis/trans d'au moins 70/30

(84) Benannte Vertragsstaaten:
**BE CH DE ES FR GB IT LI NL**

(30) Priorität: **18.12.1997 DE 19756400**

(43) Veröffentlichungstag der Anmeldung:
**30.06.1999 Patentblatt 1999/26**

(73) Patentinhaber: **BASF AKTIENGESELLSCHAFT**
**67056 Ludwigshafen (DE)**

(72) Erfinder:
- **Voit, Guido Dr.**
  **67251 Freinsheim (DE)**
- **Witzel, Tom Dr.**
  **Ota-ku, Tokyo 145-0071 (JP)**
- **Breitscheidel, Boris Dr.**
  **67117 Limburgerhof (DE)**
- **Luyken, Hermann**
  **67069 Ludwigshafen (DE)**
- **Ross, Karl-Heinz Dr.**
  **67269 Grünstadt (DE)**
- **Wahl, Peter Dr.**
  **68526 Ladenburg (DE)**

(56) Entgegenhaltungen:
**EP-A- 0 449 089**
**EP-A- 0 659 733**
**EP-A- 0 729 937**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 926 130 B1

## Beschreibung

Die vorliegende Erfindung betrifft Verfahren zur Herstellung von 3-Aminomethyl-3,5,5-trimethyl-cyclohexylamin mit einem cis/trans-Isomerenverhältnis von mindestens 70/30 durch

a) Iminierung von 3-Cyano-3,5,5-trimethyl-cyclohexanon mit Ammoniak in Gegenwart eines Iminierungskatalysators bei Temperaturen von 20 bis 150 °C und Drücken von 1,5 bis 30 MPa zu 3-Cyano-3,5,5-trimethyl-cyclohexanon-imin und anschließende

b) Hydrierung des 3-Cyano-3,5,5-trimethyl-cyclohexanon-imins in Gegenwart von Ammoniak an Katalysatoren enthaltend Kupfer und/oder ein Metall der Gruppe VIII des Periodensystems bei Temperaturen von 80 bis 160 °C und Drücken von 5 bis 30 MPa.

3-Aminomethyl-3,5,5-trimethyl-cyclohexylamin (Isophorondiamin, IPDA) ist ein wichtiges Zwischenprodukt für Polyamide und Epoxidharze und zur Herstellung des entsprechenden Isophorondiisocyanats (IPDI), das als Komponente in Polyurethanen Verwendung findet.

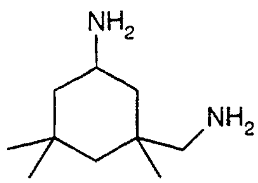

IPDA

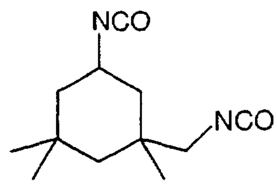

IPDI

Das IPDA-Molekül besitzt zwei asymmetrisch substituierte C-Atome und kommt daher in zwei diastereomeren Formen, dem cis- und dem trans-Isomer, wie untenstehendes Schema verdeutlicht, vor.

Bei der Herstellung von IPDA aus 3-Cyano-3,5,5-trimethyl-cyclohexanon (Isophoronnitril, IPN) durch die Schritte Iminierung und anschließende Hydrierung in Gegenwart von Ammoniak wird das resultierende cis/trans-Isomerenverhältnis des IPDAs erst im Hydrierschritt festgelegt (siehe Schema):

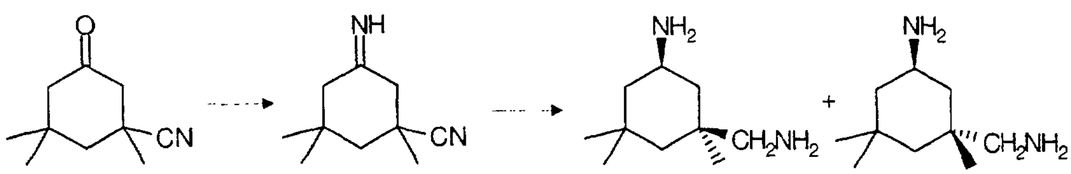

IPN IPNI cis-IPDA trans-IPDA

Dieses Isomerenverhältnis ist von sehr hoher technischer Bedeutung, da gemäß der Lehre von DE-A-42 11 454 die beiden Isomeren in ihrer Anwendung als Komponenten in Polyadditionsharzen, wie zum Beispiel Epoxidharzen, unterschiedliche Reaktivitäten aufweisen. Im Hinblick auf Reaktionsgeschwindigkeiten und Produkteigenschaften wird daher IPDA mit einem Anteil des cis-Isomeren von 75 % bevorzugt und marktgängiges IPDA (und damit auch das hieraus hergestellte IPDI) besitzt deshalb ein cis/trans-Isomerenverhältnis von 75/25.

Aus der EP-A-449 089 ist ein Verfahren zur Herstellung von IPDA aus IPN bekannt. Man setzt in einer ersten Stufe IPN mit Ammoniak an aciden Metalloxiden als Iminierungskatalysator zum 3-Cyano-3,5,5-trimethyl-cyclohexanon-imin (Isophoronnitril-imin, IPNI) um und hydriert dann in einem zweiten Schritt nach Zugabe von Wasserstoff in Gegenwart von Ammoniak an bekannten Hydriermetallen, bevorzugt Cobalt und/oder Ruthenium, zum IPDA.

Das in der EP-A-449 089 beschriebene Verfahren ermöglicht die Herstellung von IPDA aus IPN mit - im Vergleich zu vorbeschriebenen Verfahren - hoher Raum-Zeit-Ausbeute und hoher chemischer Ausbeute. So sind bei Einsatz von Aluminiumoxid oder Titandioxid in der Iminierung und hochaktiven Cobalt-Katalysatoren für die Hydrierung, wie sie z.B. in DE-A-43 25 847 beschrieben sind, IPDA-Ausbeuten von 98 % erreichbar. Der cis-IPDA-Anteil im Reaktionsaustrag liegt dabei jedoch nur bei 60 bis 66 %.

Aus der EP-A-729 937 (Seite 3, Vergleichsbeispiel A nach EP-A-449 089) ist weiterhin bekannt, daß durch Zugabe von NaOH in die Hydrierung an einem Co-Katalysator in einstufiger Hydrierfahrweise die IPDA-Ausbeute von 92 auf 97% angehoben werden kann, hierbei jedoch das Isomerenverhältnis von 68/32 auf 60/40 abfällt.

Aus der EP-A-659 734 ist es bekannt, daß es möglich ist, IPDA mit einem cis/trans-Isomerenverhältnis oberhalb desjenigen, das unter Verwendung eines Cobalt-Katalysators erhältlich ist, herzustellen, wenn man die aminierende Hydrierung von IPN in Gegenwart eines Ruthenium-Katalysators und eines optional nachgeschalteten Cobalt-Katalysators in Rieselbettreaktoren durchführt (vgl. loc. cit., Seite 5, Zeilen 36-40).

Nachteilig an diesem Verfahren sind jedoch schlechte IPDA-Ausbeuten von nur 82 bis 87 % (loc. cit., Beispiele 9 bis 11).

Zur Erzielung von cis/trans-Isomerenverhältnissen von mindestens 70/30 in Reaktionsausträgen von IPDA-Synthesen ausgehend von IPN wurden zwei weitere unterschiedliche technische Verfahren beschrieben, deren Lehre im wesentlichen darin besteht, die Hydrierstufe in zwei Schritten und bei unterschiedlichen Temperaturen durchzuführen:

Die EP-A-729 937 beschreibt die Herstellung von IPDA in Ausbeuten von > 96 % mit einem erhöhten cis-Isomerenanteil (> 67 %) aus IPN, indem man in drei räumlich voneinander getrennten Reaktionsräumen

a) das IPN mit $NH_3$ an aciden Metalloxidkatalysatoren (= Iminierungskatalysator) bei Temperaturen von 20 bis 150 °C und Drükken von 5 bis 30 MPa umsetzt,

b) die entstandenen Reaktionsprodukte in einem zweiten Reaktionsraum mit Wasserstoff in Gegenwart von $NH_3$ an Hydrierkatalysatoren bei Temperaturen von 50 bis 100 °C und Drücken von 5 bis 30 MPa hydriert und

c) die entstandenen Reaktionsprodukte in einem dritten Reaktionsraum in Gegenwart von Wasserstoff und $NH_3$ an Hydrierkatalysatoren bei Temperaturen von 110 bis 160 °C und Drücken von 15 bis 30 MPa hydriert.

Nachteilig an diesem Verfahren ist der apparative Aufwand durch die erforderlichen zwei nacheinandergeschalteten Hydrier-Druckreaktoren.

Die EP-A-659 733 offenbart einen Weg zur Steuerung des cis/trans-Isomerenverhältnisses bei der Herstellung von IPDA durch aminierende Hydrierung von IPN in Gegenwart von Ammoniak, $H_2$ und einem Hydrierkatalysator, indem man die Umsetzung in zwei Stufen bei unterschiedlichen Temperaturen, nämlich zunächst bei 10 bis 90 °C und dann bei 90 bis 150 °C, bei einer Temperaturdifferenz zwischen beiden Stufen von mindestens 30 °C, durchführt, wobei die Kontaktzeit in der ersten Stufe kürzer ist als in der zweiten. Durch Absenken der Temperatur in der ersten Stufe erhöht sich das cis/trans-Isomerenverhältnis. Gemäß den Beispielen der EP-A-659 733 sind bei einer Reaktionstemperatur des zweiten Reaktors von 120 °C und einer um mindestens 30 °C niedrigeren Temperatur im ersten Reaktor cis/trans-Isomerenverhältnisse von mindestens 70/30 erreichbar.

Nachteilig an diesem Verfahren ist wiederum der apparative Aufwand durch die erforderlichen zwei nacheinandergeschalteten Hydrier-Druckreaktoren.

Die Aufgabe bestand daher in der Suche nach einem alternativen, verfahrenstechnisch einfacheren und wirtschaftlichen Verfahren zur IPDA-Herstellung, das sowohl hohe chemische Ausbeuten und hohe Raum-Zeit-Ausbeuten als auch einen erhöhten Anteil an cis-IPDA liefert.

Demgemäß wurde ein neues und verbessertes Verfahren zur Herstellung von IPDA mit einem cis/trans-Isomerenverhältnis von mindestens 70/30 durch

a) Iminierung von 3-Cyano-3,5,5-trimethyl-cyclohexanon mit Ammoniak in Gegenwart eines Iminierungskatalysators bei Temperaturen von 20 bis 150 °C und Drücken von 1,5 bis 30 MPa zu 3-Cyano-3,5,5-trimethyl-cyclohexanon-imin und anschließende
b) Hydrierung des 3-Cyano-3,5,5-trimethyl-cyclohexanon-imins in Gegenwart von Ammoniak an Katalysatoren enthaltend Kupfer und/oder ein Metall der Gruppe VIII des Periodensystems bei Temperaturen von 80 bis 160 °C und Drücken von 5 bis 30 MPa

gefunden, welches dadurch gekennzeichnet ist,
daß man die katalytische Hydrierung des 3-Cyano-3,5,5-trimethylcyclohexanon-imins in Gegenwart einer Säure durchführt, wobei die Menge dieser Säure einer Säurezahl von 0,1 bis 2, bezogen auf eingesetztes IPN, entspricht.

Das erfindungsgemäße Verfahren läßt sich wie folgt durchführen:

Stufe a)

In der ersten Verfahrensstufe setzt man IPN mit überschüssigem Ammoniak in Gegenwart eines Iminierungskatalysators bei Temperaturen von 20 bis 150 °C, vorzugsweise von 30 bis 130 °C, besonders bevorzugt von 50 bis 100 °C, und Drücken von 1,5 bis 30 MPa, vorzugsweise von 10 bis 25 MPa, zu 3-Cyano-3,5,5-trimethyl-cyclohexanon-imin (IPNI) um.

Hierbei wird ein IPN eingesetzt, daß entweder eine Säurezahl (SZ) im Bereich von 0 bis < 0,1 aufweist, oder eine SZ im Bereich von 0,1 bis 2, bevorzugt von 0,2 bis 1, besitzt.

Die Säurezahl (SZ) gibt an, wieviel Milligramm Kaliumhydroxid zur Neutralisation der in 1 g Substanz vorhandenen freien Säuren notwendig sind (siehe z.B.: Europäisches Arzneibuch, 3. Ausgabe, Seite 67, Deutscher Apotheker Verlag Stuttgart - Govi Verlag - Pharmazeutischer Verlag, 1997). Die SZ berechnet sich nach

$$SZ = 5{,}610 \cdot n \,/\, m\ ,$$

mit $m$ = Einwaage der Substanz in Gramm
und $n$ = Verbrauch an 0,1 molarer Kaliumhydroxid-Lösung in ml bei der Titration.

IPN besitzt, da es keine sauren Gruppierungen im Molekül besitzt, gemäß obiger Definition der Säurezahl, eine SZ von Null.

Durch den Zusatz von entsprechenden Mengen einer Säure zum IPN, läßt sich eine Säurezahl im Bereich von 0,1 bis 2 einstellen. Beispielsweise wird für IPN, das pro 100 g mit 0,129 g 2-Ethylhexansäure vermischt wurde, durch Titration eine SZ von 0,5 gemessen.

Als Säuren, die im erfindungsgemäßen Verfahren eingesetzt werden können, sind alle Säuren geeignet, die nach entsprechender Zugabe zu IPN, eine Einstellung der IPN-Säurezahl im Bereich von 0,1 bis 2 ermöglichen. Geeignet sind Lewis- und Brönsted-Säuren, bevorzugt Brönsted-Säuren und Gemische hiervon, besonders bevorzugt Brönsted-Säuren mit einem pKs-Wert kleiner 6, ganz besonders bevorzugt organische Brönsted-Säuren, wie z.B. Monocarbonsäuren und Dicarbonsäuren.

Geeignete Säuren sind z.B. Lewis-Säuren, wie Aluminiumtrichlorid, Zinkdichlorid, Bortrifluorid, Bortrifluorid-etherat, anorganische Säuren, wie Phosphorsäure, phosphorige Säure und Schwefelsäure, Sulfonsäuren, wie Methansulfonsäure und p-Toluolsulfonsäure, und $C_1$- bis $C_{20}$-Carbonsäuren, wie Ameisensäure, Essigsäure, Methoxyessigsäure, Propionsäure, Capronsäure, Laurinsäure, Benzoesäure, Phthalsäure, Phenylessigsäure, 2-Ethylhexansäure, Bernsteinsäure, Glutarsäure, Adipinsäure, Korksäure, Sebacinsäure, bevorzugt Essigsäure, 2-Ethylhexansäure und Adipinsäure, besonders bevorzugt 2-Ethylhexansäure.

Als Iminbildungskatalysatoren kommen beispielsweise feste Brönsted- oder Lewis-Säuren in Betracht wie sie in der EP-A-449 089 (Seite 2, Spalte 2, Zeilen 10-18), in der EP-A-42 119 und in 'Studies in surface science and catalysis, Vol. 51, S. 1 ff. (Elsevier, 1989): K. Tanabe et al., New Solid Acids - their catalytic properties' beschrieben worden sind. Beispielhaft seien hier acide Metalloxidkatalysatoren, wie Aluminiumoxid, Titandioxid und Zirkondioxid, oder mit Ammonium-Ionen beladene anorganische oder organische Ionenaustauscher, wie Zeolithe oder sulfonierte Copolymerisate aus Styrol und Divinylbenzol (z.B. der Marken Lewatit®, Amberlite®) oder Austauscher auf Basis Siloxan (z.B. der Marke Deloxan®), genannt.

Bei Verwendung von aciden Metalloxiden oder Ionenaustauschern als Iminierungskatalysator hält man eine Katalysatorbelastung von 0,01 bis 10, vorzugsweise von 0,05 bis 7, besonders bevorzugt von 0,1 bis 5 kg IPN pro kg Katalysator und Stunde ein.

Pro Mol IPN setzt man bei der Iminierung zweckmäßig, aber nicht zwingend, 5 bis 500 Mol $NH_3$, bevorzugt 10 bis 400 Mol $NH_3$, besonders bevorzugt 20 bis 300 Mol $NH_3$ ein.

Die Iminierung des IPNs kann auch in Anwesenheit eines Lösungsmittels, wie z.B. Alkanolen oder Tetrahydrofuran durchgeführt werden, bevorzugt arbeitet man aber ohne Zusatz eines Lösungsmittels.

Die Iminierung wird bevorzugt kontinuierlich durchgeführt, z.B. in Druckbehältern oder Druckbehälterkaskaden. Nach einer besonders bevorzugten Ausführungsform werden IPN und $NH_3$ durch einen Rohrreaktor geleitet, in dem der Iminierungskatalysator in Form eines festen Bettes angeordnet ist.

Stufe b)

Für den Fall, daß in der Iminierungsstufe (Stufe a), siehe oben) ein IPN eingesetzt wurde, das eine Säurezahl von 0,1 bis 2 besaß, wird das in der Stufe a) erhaltene Reaktionsprodukt direkt in der weiteren Stufe b) (siehe weiter unten) eingesetzt.

Wurde in der Stufe a) ein IPN eingesetzt, das eine Säurezahl von 0 bis < 0,1 besaß, so wird dem in der Stufe a) erhaltenen Reaktionsaustrag eine Säure zugegeben, und zwar in derjenigen Menge, die einer Säurezahl von 0,1 bis 2, bevorzugt 0,2 bis 1, bezogen auf eingesetztes IPN, entspricht. Für die Art der einsetzbaren und bevorzugten Säuren gilt das unter a) (Iminierungsstufe) Gesagte. Bei einer kontinuierlichen Durchführung der Stufe b) (siehe weiter unten) kann auch die Säure dem Reaktorzulauf kontinuierlich zugeführt werden.

Der in der Iminierungsstufe erhaltene Reaktionsaustrag, der aufgrund der oben beschriebenen Maßnahmen eine Säure in der Menge enthält, die einer Säurezahl von 0,1 bis 2, bevorzugt 0,2 bis 1, bezogen auf eingesetztes IPN, entspricht, wird in einer zweiten Stufe mit 3 bis 10.000 Moläquivalenten (bezogen auf Isophoronnitril-imin) Wasserstoff, bevorzugt 4,5 bis 100 Moläquivalenten $H_2$, gegebenenfalls nach Zufuhr von weiterem Ammoniak, einer katalytischen Hydrierung unterzogen.

Die Hydrierung erfolgt bei Reaktionstemperaturen von 80 bis 160 °C, beispielsweise bei 100, 120, 130, 140 oder 150 °C, bevorzugt bei 120 bis 150 °C, und einem Druck von 5 bis 30 MPa, bevorzugt 10 bis 25 MPa.

Als Hydrierkatalysatoren können prinzipiell alle Hydrierkatalysatoren eingesetzt werden, die Nickel, Cobalt, Eisen, Kupfer, Ruthenium und/oder andere Metalle der VIII. Nebengruppe des Periodensystems enthalten. Bevorzugt verwendet man ruthenium- und/oder cobalt- und/oder nickelhaltige Katalysatoren. Besonders bevorzugt sind Ruthenium- und Cobalt-Katalysatoren und Gemische hiervon. Die katalytisch aktiven Metalle können als Vollkontakte oder auf Trägern eingesetzt werden. Als solche Träger kommen z.B. Aluminiumoxid, Titandioxid, Zirkondioxid, Zinkoxid oder Magnesiumoxid/Aluminiumoxid in Frage, bevorzugt werden Träger mit basischen Komponenten, wie Oxide und Hydroxide von Alkali- und Erdalkalimetallen. Besonders bevorzugt sind Vollkontakte, wie sie z.B. aus der DE-A-43 25 847 und der EP-A-742 045 bekannt sind, die basische Komponenten wie Oxide oder Hydroxide von Alkali und Erdalkalimetallen enthalten.

Die Katalysatorbelastungen bei kontinuierlicher Verfahrensweise (in kg Reaktorzulauf / [kg Katatysator • Stunde]), liegen zweckmäßigerweise im Bereich von 0,01 bis 5 kg/[kg • h], bevorzugt bei 0,02 bis 2,5 kg/[kg • h], besonders bevorzugt bei 0,05 bis 2 kg/ [kg • h].

Die Hydrierung wird vorzugsweise in flüssigem Ammoniak durchgeführt. Pro Mol 3-Cyano-3,5,5-trimethylcyclohexanon-imin (IPNI) setzt man 5 bis 500 Mol $NH_3$, bevorzugt 10 bis 400 Mol, besonders bevorzugt 20 bis 300 Mol, ein. Zweckmäßigerweise wählt man mindestens dasjenige $NH_3$-Angebot, das bei der vorgelagerten Herstellung von IPNI aus IPN (Stufe a)) eingestellt wurde. Der $NH_3$-Anteil kann jedoch auch vor der Hydrierung durch Zugabe von zusätzlichem $NH_3$ auf einen gewünschten Wert erhöht werden.

Die Hydrierung von 3-Cyano-3,5,5-trimethyl-cyclohexanon-imin (IPNI) in Gegenwart von $NH_3$ führt man bevorzugt kontinuierlich, z.B. in druckfesten Rührbehältern oder in einer Rührbehälterkaskade, durch. In einer besonders bevorzugten Ausführungsform werden Rohrreaktoren eingesetzt, in denen die Hydrierung in Sumpf- oder Rieselfahrweise an einen ein fest angeordneten Katalysatorbett erfolgt.

Falls der Reaktoraustrag der Hydrierstufe (Stufe b) noch nicht vollständig umgesetzte Komponenten, wie z. B. 3-Cyano-3,5,5-trimethyl-cyclohexylamin ('Aminonitril'), welches vom IPDA destillativ nur sehr aufwendig abtrennbar ist, enthält, kann er in einer dritten Stufe (Stufe c)) in Gegenwart von Wasserstoff und Ammoniak an den unter Stufe b) beschriebenen Hydrierkatalysatoren bei Reaktionstemperaturen von 110 bis 160 °C, beispielsweise 120, 130, 140 oder 150 °C, und Drücken von 5 bis 30 MPa, bevorzugt 15 bis 25 MPa, umgesetzt werden. Zweckmäßigerweise wählt man das Ammoniak- und Wasserstoffangebot so, wie es sich am Reaktoraustritt der Stufe b) ergibt.

Der Reaktor der Stufe c), bei dem es sich in einer bevorzugten Ausführungsform um einen Rohrreaktor mit Katalysatorfestbett handelt, kann deutlich kleiner als derjenige der Stufe b) sein. Beispielsweise besitzt der Reaktor der Stufe c) ein Leervolumen, das 20 bis 40 % des Leervolumens des Reaktors der Stufe b) entspricht.

Nach der Hydrierung werden überschüssiges Ammoniak und gegebenenfalls Wasserstoff, gegebenenfalls unter Druck, aus dem Reaktoraustrag abgetrennt. Das so erhaltene rohe IPDA mit einem cis/trans-Isomerenverhältnis von mindestens 70/30 läßt sich durch eine fraktionierende Rektifikation rein isolieren.

Beispiel

In zwei hintereinandergeschalteten Rohrreaktoren, der erste (= Iminierungsreaktor) gefüllt mit 400 ml gamma-Aluminiumoxid (4-mm-Stränge), der zweite (= Hydrierreaktor) gefüllt mit 800 ml eines Cobaltkatalysators (4-mm-Stränge) mit einem Cobalt-Gehalt von 90 %, wie er in der EP-A-742 045 (Seite 4) beschrieben wurde, wurden stündlich 160 g Isophoronnitril (IPN) mit einer Säurezahl von < 0,1 und 480 g flüssiges $NH_3$ zugefahren. Vor dem Hydrierreaktor wurden zusätzlich 500 Nl/h Wasserstoff zugefahren. (Anmerkung: Nl = Normliter = auf Normalbedingungen umgerechnete Volumenangabe). Der Druck in beiden Reaktoren betrug 25 MPa, die Reaktionstemperatur betrug im ersten Reaktor 85 °C und im zweiten Reaktor 135 °C.

Eine gaschromatographische Analyse des Reaktionsaustrags zeigte ein cis/trans-IPDA-Isomerenverhältnis von 66/34.

Nach einer Laufzeit von 1130 h wurde im eingesetzten IPN durch Zugabe einer entsprechende Menge 2-Ethylhexansäure eine Säurezahl von 0,5 (g KOH/kg IPN) eingestellt. Daraufhin stieg das cis/trans-IPDA-Isomerenverhältnis im Reaktoraustrag auf 75/25, bei einer IPDA-Ausbeute von 92 %.

## Patentansprüche

1. Verfahren zur Herstellung von 3-Aminomethyl-3,5,5-trimethylcyclohexylamin mit einem cis/trans-Isomerenverhältnis von mindestens 70/30 durch

   a) Iminierung von 3-Cyano-3,5,5-trimethyl-cyclohexanon mit Ammoniak in Gegenwart eines Iminierungskatalysators bei Temperaturen von 20 bis 150 °C und Drücken von 1,5 bis 30 MPa zu 3-Cyano-3,5,5-trimethyl-cyclohexanon-imin und anschließende

   b) Hydrierung des 3-Cyano-3,5,5-trimethyl-cyclohexanon-imins in Gegenwart von Ammoniak an Katalysatoren enthaltend Kupfer und/oder ein Metall der Gruppe VIII des Periodensystems bei Temperaturen von 80 bis 160 °C und Drücken von 5 bis 30 MPa,

   **dadurch gekennzeichnet, daß** man die katalytische Hydrierung des 3-Cyano-3,5,5-trimethyl-cyclohexanon-imins in Gegenwart einer Säure durchführt, wobei die Menge dieser Säure einer Säurezahl von 0,1 bis 2, bezogen auf eingesetztes 3-Cyano-3,5,5-trimethyl-cyclohexanon, entspricht.

2. Verfahren nach Anspruch 1, **dadurch gekennzeichnet, daß** man die katalytische Hydrierung des 3-Cyano-3,5,5-trimethyl-cyclohexanon-imins in Gegenwart einer Säure durchführt, wobei die Menge dieser Säure einer Säurezahl von 0,2 bis 1, bezogen auf eingesetztes 3-Cyano-3,5,5-trimethyl-cyclohexanon, entspricht.

3. Verfahren nach den Ansprüchen 1 oder 2, **dadurch gekennzeichnet, daß** es sich bei der Säure um eine Brönsted-Säure oder Gemische hiervon handelt.

4. Verfahren nach Anspruch 3, **dadurch gekennzeichnet, daß** die Brönsted-Säure einen pKs-Wert kleiner 6 besitzt.

5. Verfahren nach Anspruch 3, **dadurch gekennzeichnet, daß** es sich bei der Säure um eine organische Brönsted-Säure handelt.

6. Verfahren nach Anspruch 5, **dadurch gekennzeichnet, daß** als Säure eine Mono- oder Dicarbonsäure oder ein Gemisch hiervon eingesetzt wird.

7. Verfahren nach Anspruch 6, **dadurch gekennzeichnet, daß** als Säure 2-Ethylhexansäure eingesetzt wird.

## Claims

1. A process for the preparation of 3-aminomethyl-3,5,5-trimethylcyclohexylamine having a cis/trans isomer ratio of at least 70:30 by

   a) imination of 3-cyano-3,5,5-trimethylcyclohexanone with ammonia in the presence of an imination catalyst at temperatures of from 20 to 150°C and pressures of from 1.5 to 30 MPa to form 3-cyano-3,5,5-trimethylcyclohexanone imine followed by

   b) hydrogenation of the 3-cyano-3,5,5-trimethylcyclohexanone imine in the presence of ammonia over a catalyst containing copper and/or a Group VIII metal at a temperature of from 80° to 160°C. and under a pressure of from 5 to 30 MPa,

   wherein the catalytic hydrogenation of the 3-cyano-3,5,5-trimethylcyclohexanone imine is carried out in the presence of an acid used in an amount such as to give an acid number of from 0.1 to 2, based on 3-cyano-3,5,5-trimethylcyclohexanone used.

2. A process as defined in claim 1, wherein the catalytic hydrogenation of 3-cyano-3,5,5-trimethylcyclohexanone imine is carried out in the presence of an acid used in an amount such as to give an acid number of from 0.2 to 1, based on 3-cyano-3,5,5-trimethylcyclohexanone used.

3. A process as defined in claim 1 or 2, wherein the acid used is a Broenstedt acid or a mixture of Broenstedt acids.

4. A process as defined in claim 3, wherein the Broenstedt acid possesses a pKa value of less than 6.

5. A process as defined in claim 3, wherein the acid used is an organic Broenstedt acid.

6. A process as defined in claim 5, wherein the acid used is a mono- or dicarboxylic acid or a mixture thereof.

7. A process as defined in claim 6, wherein the acid used is 2-ethylhexanoic acid.

## Revendications

1. Procédé de préparation de 3-aminométhyl-3,5,5-triméthylcyclohexylamine, ayant un rapport entre isomères cis/trans d'au moins 70/30, par

   a) imination de 3-cyano-3,5,5-triméthyl-cyclohexanone avec de l'ammoniac en présence d'un catalyseur d'imination à des températures de 20 à 150°C et sous des pressions de 1,5 à 30 MPa, pour donner la 3-cyano-3,5,5-triméthyl-cyclohexanone-imine, puis

   b) hydrogénation de la 3-cyano-3,5,5-triméthyl-cyclohexanone-imine en présence d'ammoniac sur des catalyseurs contenant du cuivre et/ou un métal du groupe VIII du Tableau Périodique à des températures de 80 à 160°C et sous des pressions de 5 à 30 MPa,

   **caractérisé en ce qu'**on met en oeuvre l'hydrogénation catalytique de la 3-cyano-3,5,5-triméthyl-cyclohexanone-imine en présence d'un acide, la quantité de cet acide correspondant à un indice d'acide de 0,1 à 2 par rapport à la 3-cyano-3,5,5-triméthyl-cyclohexanone utilisée.

2. Procédé selon la revendication 1, **caractérisé en ce qu'**on met en oeuvre l'hydrogénation catalytique de la 3-cyano-3,5,5-triméthylcyclohexanone-imine en présence d'un acide, la quantité de cet acide correspondant à un indice d'acide de 0,2 à 1 par rapport à la 3-cyano-3,5,5-triméthyl-cyclohexanone utilisée.

3. Procédé selon les revendications 1 ou 2, **caractérisé en ce que**, pour ce qui concerne l'acide, il s'agit d'un acide de Brönsted ou de mélange de ceux-ci.

4. Procédé selon la revendication 3, **caractérisé en ce que** l'acide de Brönsted a un pKs inférieur à 6.

5. Procédé selon la revendication 3, **caractérisé en ce que**, pour ce qui concerne l'acide, il s'agit d'un acide de Brönsted organique.

6. Procédé selon la revendication 5, **caractérisé en ce qu'**on utilise en tant qu'acide un dérivé d'un acide mono- ou dicarboxylique ou un mélange de ceux-ci.

7. Procédé selon la revendication 6, **caractérisé en ce qu'**on utilise en tant qu'acide l'acide 2-éthylhexanoïque.